# EUROPEAN PATENT SPECIFICATION

(11) **EP 3 400 028 B1**
(45) Date of publication and mention of the grant of the patent: **07.01.2026**
(21) Application number: 17735920.5
(22) Date of filing: 02.01.2017
(51) Int. Cl.: A61L 27/38, A61L 27/36, A61L 27/24, A61L 27/12, A61L 27/54, A61P 17/00, A61P 25/28, A61P 29/00, A61P 31/04, A61B 17/06, A61B 17/00, A61L 17/00

(54) **SKIN-AUGMENTING SURGICAL SUTURES**
HAUTVERBESSERNDE CHIRURGISCHE NAHTMATERIALIEN
SUTURES CHIRURGICALES D'AUGMENTATION DERMIQUE

(30) Priority: 05.01.2016 IL 24346116
(43) Date of publication of application: 14.11.2018
(73) Proprietor: Amir, Avraham, 40600 Tel Mond (IL)
(72) Inventor: Amir, Avraham, 40600 Tel Mond (IL)
(74) Representative: Pearl Cohen Zedek Latzer Baratz UK LLP
(86) International application number: PCT/IL2017/050006
(87) International publication number: WO 2017/118972

(56) References cited:
- WO-A1-2010/028025
- WO-A1-87/07495
- GB-A- 386 161
- US-A1- 2008 248 079
- US-A1- 2013 018 415
- US-A1- 2015 327 972
- US-B2- 7 329 271

## Description

### FIELD OF THE INVENTION

The present invention relates to biocompatible surgical sutures comprising dermal fillers, and methods of use thereof. In particular, the sutures of the present invention are configured to temporarily accommodated dermal fillers which are aimed for the filling of undesired lines, wrinkles, depressed scars and folds of a subject's skin and restoring youthful fullness to the skin.

### BACKGROUND OF THE INVENTION

Skin is composed of the epidermis and the dermis. Below these layers lies the hypodermis, which is not usually classified as a layer of skin. The hypodermis is also commonly referred to as subcutaneous fat layer, sub-cutis or subcutaneous tissue. The outermost epidermis is made up of stratified squamous epithelium with an underlying basement membrane. It contains no blood vessels, and is nourished by diffusion from the dermis. The epidermis is mainly composed of keratinocytes, with melanocytes and Langerhans cells also present. This layer of skin functions as a barrier between the body and the external environment, keeping water in the body and preventing penetration of harmful chemicals and pathogens.

The dermis lies below the epidermis and contains a number of structures including blood vessels, nerves, hair follicles, smooth muscle, glands and lymphatic tissue. The dermis (or corium) is typically 0.1-3 mm thick and is the major component of human skin. It is composed of a network of connective tissue, predominantly collagen fibrils providing support and elastin fibers providing flexibility. The main cell types composing the dermis are fibroblasts, adipocytes (fat storage) and macrophages.

The hypodermis lies below the dermis and is important for attaching the skin to the underlying bone and muscle as well as supplying it with blood vessels and nerves. The hypodermis is made up of loose connective tissue and elastin and contains fibroblasts, macrophages and adipocytes. The adipocytes play a major role in the fat storage function of the hypodermis. The fat serves as a filling material and as insulation of the body from the external environment.

Facial aging occurs as the result of several factors, among them are inherent changes within the skin, effects of gravity, activity of facial muscles leading to the formation of dynamic lines, skin loss or shift, bone loss, loss of tissue elasticity and exposure to harsh environmental conditions, particularly the sun or ultraviolet radiation and pollutants. When the skin ages, the epidermis begins to thin, causing the junction with the dermis to flatten. Collagen decreases as a person ages and the bundles of collagen, which gives the skin turgor, become looser and lose strength. When the skin loses elasticity, it is less able to resist stretching. Due to gravity, muscle pull and tissue changes, the skin begin to wrinkle. Water loss and breakdown of bonds between cells also reduce the barrier function of the skin, which can cause the skin's pore size to increase.

A wrinkle is a fold, ridge or crease in the skin. Skin wrinkles typically appear as a result of aging processes such as glycation, habitual sleeping positions, or loss of body mass. Age wrinkling in the skin is promoted by habitual facial expressions, aging, sun damage, smoking, poor hydration, and various other factors.

There have been efforts to develop and use compositions to correct defects in skin, such as scars and wrinkles, or to augment the tissue of a subject in order to improve the appearance of the skin, particularly facial skin. Wrinkles of different etiologies and of different severities are conventionally treated by local injections of Tretinoin (retinoic acid), glycosaminoglycans, botulinum toxin and/or dermal fillers, or by laser resurfacing techniques.

Dermal fillers are usually injectable products, frequently used to correct wrinkles and other depressions in the skin. They are designed to enable injection into the skin for purposes of improving the appearance of the skin. The most common products are based on hyaluronic acid and calcium hydroxyapatite.

Currently, there are dozens of known dermal filling agents for skin augmentation which include autologous implantable materials, allogeneic products, xenogeneic products and synthetically derived products. Available dermal fillers comprise biodegradable natural substances (such as collagen, gelatin, hyaluronic acid, dextran and dried acellular particulate dermal matrix), biodegradable synthetic polymers (such as poly-L-lactic acid, polyethylene oxide and carboxymethylcellulose), non-biodegradable synthetic polymers (such as polymethyl methacrylate, polyacrylamide, polyalkylimide and silicones) and combinations thereof.

Biocompatible ceramic skin augmentation materials, such as hydroxyapatite, are known to be efficient skin augmentation materials due to their properties: Hydroxyapatite (Ca₅(PO₄)₃(OH)) is a naturally occurring mineral form of calcium phosphate. Hydroxyapatite comprises the mineral constituent of bone, therefore rendering it biocompatible and non-immunogenic when introduced into the body of a subject. Of note, hydroxyapatite is biodegradable following the same metabolic pathways as bone debris resulting from common bone fractures, yet is semi-permanent, as it lasts up to 3 years when implanted into a subject's skin or sub-cutis. Moreover, when injected as small microspheres, hydroxyapatite acts as a scaffold that promotes new tissue formation similar to its surrounding environment. Inside skins such as the dermis, deposited particles of Hydroxyapatite support fibroblastic ingrowth and new collagen formation (Jacovella, P.F, Clin. Interv. Aging., 2008, 3(1): 161-174, Suchanek W. and Yoshimura M., J. Mater. Res., 1997, 13(1): 94-117).

Surgical sutures (commonly called stitches) are medical devices used to hold body tissues together after an injury or surgery. Application generally involves using a needle with an attached length of thread. A number of different shapes, sizes, and thread materials have been developed thus far. Surgeons, physicians, dentists, podiatrists, eye doctors, registered nurses and other trained nursing personnel, medics, and clinical pharmacists typically engage in suturing.

Sutures may be classified as either bio-absorbable or non-bio-absorbable depending on whether the body will naturally degrade and absorb the suture material over time. Absorbable suture materials may include for example polyglycolic acid, polylactic acid, polydioxanone, and caprolactone. They are broken down by various processes including hydrolysis (polyglycolic acid) and proteolytic enzymatic degradation. Depending on the material, the process can take from seven days to eight weeks. Absorbable sutures are used in all kinds of patients, and particularly in patients who cannot return for suture removal, in uncooperative patients (like children), in internal body tissues. In all cases, they hold the body tissues together long enough to allow healing, but disintegrate so that they do not leave foreign material or require further procedures.

US 2008/248079A1 relates to a tissue augmentation device that comprises an elongate tissue penetrating member and an amount of remodelable material; at least a portion of the elongate member is cannulated, and at least a portion of the amount of material is received within at least a portion of the cannulated portion of the elongate member.

WO 2010/028025 relates to self-swellable or self-expandable threads for soft tissue augmentation. US 2008/0188416 relates to tissue filler compositions that are alterable or removable on demand. U.S. 5,652,056 relates to substantially pure hydroxyapatite filaments.

Limitations and disadvantages of conventional and traditional anti-wrinkle approaches will become apparent to one of skill in the art, through comparison of such systems with some aspects of the present invention as set forth in the remainder of the present application with reference to the drawings. It is thus desirable to have highly efficient means for targeted, long lasting skin augmentation products.

### SUMMARY OF THE INVENTION

The invention is as defined in claims 1 to 15.

The present invention relates to biocompatible sutures, in particular, surgical sutures, which comprise biocompatible dermal fillers, useful for augmenting skin in a subject. In particular, the sutures of the invention are useful for filling undesired lines, wrinkles, depressed scars and folds of a subject's skin. According to the principles of the invention, the sutures of the present invention advantageously allow pin-point administration and targeting of metered dosages of dermal fillers into the skin of a subject, and further allow trained personal, such as plastic surgeons, to administer the suture, and therefore the filler, in substantially any spatial configuration. While the most frequently used current anti-wrinkle techniques for using fillers are limited to bolus injections of injectable fillers which form noticeable bulges on a subject's skin, the sutures and methods provided by the present disclosure are free from these limitations, and allow the filler to be evenly and/or homogenously administered as liquids, solids or semi-solids (like gels), and obtain practically any configuration during and after administration, as desired by the e.g. plastic surgeon.

According to the principles of the present invention, another advantage of a combination of flexible solid threads with a chemically-different dermal fillers is that any dermal filler which cannot, for any reason, be formulated as a thread capable of withstanding the forces and/or pressures involved in penetrating the skin surface, moving through or below the skin, and stitching the skin, can now be delivered into the skin regardless of these limitations.

The present invention provides, in one aspect, a surgical suture, comprising (i) at least one flexible solid matrix formulated in the shape of a thread, the thread having an ultimate tensile strength sufficient to pull the thread through a human skin or sub-cutis and manipulate the thread once in the skin or sub-cutis such that the integrity of the thread is not substantially compromised; the at least one flexible solid matrix being in association with (ii) at least one dermal filler, wherein the dermal filler is chemically different from the flexible solid matrix, and wherein the dermal filler gradually dissociates from the flexible solid matrix while in the skin or the sub-cutis.

In certain embodiments, the dermal filler is biocompatible. In certain embodiments, the dermal filler is absorbed within 1 to 2 years. In certain embodiments, the dermal filler promotes collagen formation. In certain embodiments, the dermal filler have minimal side-effects. In certain embodiments, the dermal filler does not produce granules or lumps. In certain embodiments, the dermal filler is inert. In certain embodiments, the dermal filler is produced in variable sizes.

In certain embodiments, at least one section of the thread is formulated in the shape of (i) a full thread, and is at least partly externally coated by the dermal filler; (ii) a grooved thread, and the groove is at least partly filled with the dermal filler; (iii) a porous thread, and is at least partly filled with the dermal filler; or (iv) a hollow thread, and is at least partly filled with the dermal filler.

In certain embodiments, the dermal filler is substantially evenly and/or homogenously distributed along at least one section of the thread.

In certain embodiments, the average diameter of the thread is 180 to 4600 microns. In certain embodiments, the average diameter of the thread is 180 to 1500 microns.

In certain embodiments, the thread is biodegradable and/or absorbable. In certain embodiments, the thread comprises a material selected from the group consisting of glycolide, polyglycolide, glycolic acid, polyglycolic acid, lactide, L-lactide, lactic acid, poly(lactic-co-glycolic acid) (PLGA), glycolide/lactide copolymer, caprolactone, glycolide/epsilon-caprolactone copolymer, dioxanone, polydioxanone, trimethyl carbonate, ethyl glycol, salts thereof, esters thereof, hydrates thereof, and any combination thereof.

In certain embodiments, the thread is non-biodegradable or non-bioabsorbable. In certain embodiments, the thread comprises a material selected from the group consisting of nylon, silk, polypropylene, polyester, stainless steel, and any combination thereof.

In certain embodiments, the dermal filler comprises at least one liquid dermal filler or at least one semi-solid dermal filler. In certain embodiments, the dermal filler is formulated as a liquid or is formulated as a semi-solid. In certain embodiments, the dermal filler comprises at least one rigid dermal filler or is formulated as solid.

In certain embodiments, the dermal filler comprises at least one rigid dermal filler. In certain embodiments, the rigid dermal filler has (i) an ultimate tensile strength of up to 300 MPa; (ii) a compressive strength of up to 1000 MPa; (iii) an elastic modulus of at least 80 GPa; (iv) a fracture toughness of up to 1 MPa.m-1/2; or (v) any combination of (i) to (iv). In certain embodiments, the rigid dermal filler has (i) an ultimate tensile strength of 40 to 300 MPa; (ii) a compressive strength of 500 to 1000 MPa; (iii) an elastic modulus of 80 to 120 GPa; (iv) a fracture toughness of 0.6 to 1 MPa.m-1/2; or (v) any combination of (i) to (iv).

In certain embodiments, the rigid dermal filler is a ceramic, a mineral or a mineraloid. In certain embodiments, the mineral is a phosphate mineral. In certain embodiments, the rigid dermal filler is brittle, malleable, ductile or sectile. In certain embodiments, the rigid dermal filler is brittle. In certain embodiments, the rigid dermal filler has Mohs hardness score of 2 to 10. In certain embodiments, the rigid dermal filler has Mohs hardness score of 5 to 10.

In certain embodiments, the rigid dermal filler is formulated in the shape of a plurality of particles. In certain embodiments, the average diameter of the particles in the plurality of particles is 5 to 500 microns. In certain embodiments, the average diameter of the particles in the plurality of particles is 10 to 100 microns.

In certain embodiments, the dermal filler is selected from the group consisting of an apatite, hyaluronic acid, polycaprolactone, polymethylmethacrylate, silicone, polylactic acid, collagen, gelatin, dermal graft, processed dermal graft, dried acellular particulate dermal matrix, fat cells, stem cells, chondrocytes, salts thereof, esters thereof, hydrates thereof, derivatives thereof, solvates thereof, and any combination thereof. Each possibility represents a separate embodiment of the invention. In certain embodiments, the dermal filler comprises hydroxyapatite, a salt thereof, an ester thereof, a hydrate thereof, a derivative thereof, a solvate thereof, and any combination thereof.

In certain embodiments, the thread is biodegradable or bioabsorbable, and wherein the dermal filler is formulated in the shape of a plurality of particles having an average diameter of 10 to 100 microns evenly distributed along at least one section of the thread.

In certain embodiments, the surgical suture of the present invention further comprises an agent which promotes the dissociation of the dermal filler from the thread while in the skin or the sub-cutis. In certain embodiments, the agent is a hygroscopic, diffusible or osmotic agent. In certain embodiments, the dermal filler is mixed or covered at least partly with an agent which stimulates diffusion or osmosis of fluids towards the dermal filler and thus release it into a skin or sub-cutis of a subject.

In certain embodiments, the surgical suture of the present invention further comprises a needle. In certain embodiments, the diameter of the needle is the same as the diameter of the surgical suture. In certain embodiments, the diameter of the needle is larger than the diameter of the surgical suture. In certain embodiments, the diameter of the needle is smaller than the diameter of the surgical suture.

In certain embodiments, the surgical suture of the present invention further comprises a cosmetic agent or a biologically-active agent. In certain embodiments, the cosmetic agent is a pigment. In certain embodiments, the biologically-active agent is selected from the group consisting of an enzyme, a drug, and a combination thereof. In certain embodiments, the enzyme is collagenase or elastase. In certain embodiments, the drug is an antibiotic agent, retinoic acid, a glycosaminoglycan (GAG), a botulinum toxin, an analgesic, an anti-scarring drug, or any combination thereof. In certain embodiments, the anti-scarring drug is a steroid.

Described but not part of the invention is a kit, comprising at least one of the surgical sutures described above.

Described but not part of the invention is a kit, comprising a plurality of different surgical sutures as described above.

In certain embodiments, the kit further comprises a needle.

The present disclosure further provides, in another aspect, a surgical suture as described above, for use in a cosmetic or therapeutic method of filling an undesired line, area, wrinkle, depressed scar or fold in skin.

In certain embodiments, the method is for reducing the depth, volume or visibility of an undesired line, area, wrinkle, depressed scar or fold in the skin of the subject. In certain embodiments, the surgical suture is to be inserted into the epidermis, the dermis, the hypodermis or the sub-cutis of the subject.

The present disclosure further provides, in another aspect, a cosmetic or therapeutic method of reducing the depth, volume or visibility of an undesired line, area, wrinkle, depressed scar or fold in the skin of a subject in need thereof, comprising the steps of (i) obtaining a surgical suture as described above; (ii) optionally, connecting a needle to the surgical suture of (i); (iii) optionally, performing local anesthesia in the undesired line, area, wrinkle, depressed scar or fold in the skin of the subject; and (iv) stitching the undesired line, area, wrinkle, depressed scar or fold in the skin of the subject with the surgical suture of (i) or (ii).

In certain embodiments, the stitching step is performed along the undesired line, area, wrinkle, depressed scar or fold. In certain embodiments, the stitching step is repeated until the undesired line, area, wrinkle, depressed scar or fold is substantially ameliorated or no longer visible. In certain embodiments, the surgical suture comprises at least a segment composed of a non-biodegradable or non-bioabsorbable thread, the method further comprises the step of removing the segment from the body of the subject. In certain embodiments, the step of removing the segment from the body of the subject is affected few hours to few weeks after the stitching.

In certain embodiments, the diameter of the suture is predetermined according to the size, depth or volume of the line, area, wrinkle, depressed scar or fold to be filled. In certain embodiments, the content of the dermal filler in the suture is predetermined according to the size, depth or volume of the line, area, wrinkle, depressed scar or fold to be filled.

The present disclosure further provides, in another aspect, a cosmetic or therapeutic method of forming a filamentous structure of at least one dermal filler in a body of a subject in need thereof, comprising the steps of (i) obtaining a surgical suture as described above; (ii) optionally, connecting a needle to the surgical suture of (i); and (iii) placing the surgical suture of (i) or (ii) within the body of the subject as to form the filamentous structure within the body of the subject.

In certain embodiments, the average diameter of the filamentous structure throughout its length is 0.1 to 10 mm. In certain embodiments, the average diameter of the filamentous structure throughout its length is 1 to 5 mm.

The present disclosure further provides, in another aspect, a cosmetic or therapeutic method of introducing an implant into a body of a subject in need thereof, comprising the steps of (i) obtaining a surgical suture as described above; (ii) forming the surgical suture of (i) in the shape of the implant; (iii) optionally, connecting a needle to the implant to allow fixation of the implant under the skin or under a mucous membrane of the subject; and (iv) placing the implant within the body of the subject as needed.

In certain embodiments, the dermal filler is selected from the group consisting of collagen, gelatin, dermal graft, processed dermal graft, dried acellular particulate dermal matrix, fat cells, stem cells, chondrocytes, fibroblasts, and any combination thereof.

In certain embodiments, the surgical sutures are different by a feature selected from the group consisting of (i) the shape of the thread, (ii) the average diameter of the thread, (iii) the material of the thread, (iv) the rigidity of the dermal filler, (v) the dermal filler, (vi) the shape of the dermal filler, (vii) the size of the dermal filler, (viii) the amount of dermal filler, and (ix) and combination of (i) to (viii). Each possibility represents a separate embodiment of the invention.

Further embodiments and the full scope of applicability of the present invention will become apparent from the detailed description given hereinafter. However, it should be understood that the detailed description and specific examples, while indicating some embodiments of the invention, are given by way of illustration only, since various changes and modifications within the spirit and scope of the invention will become apparent to those skilled in the art from this detailed description.

### BRIEF DESCRIPTION OF THE DRAWINGS

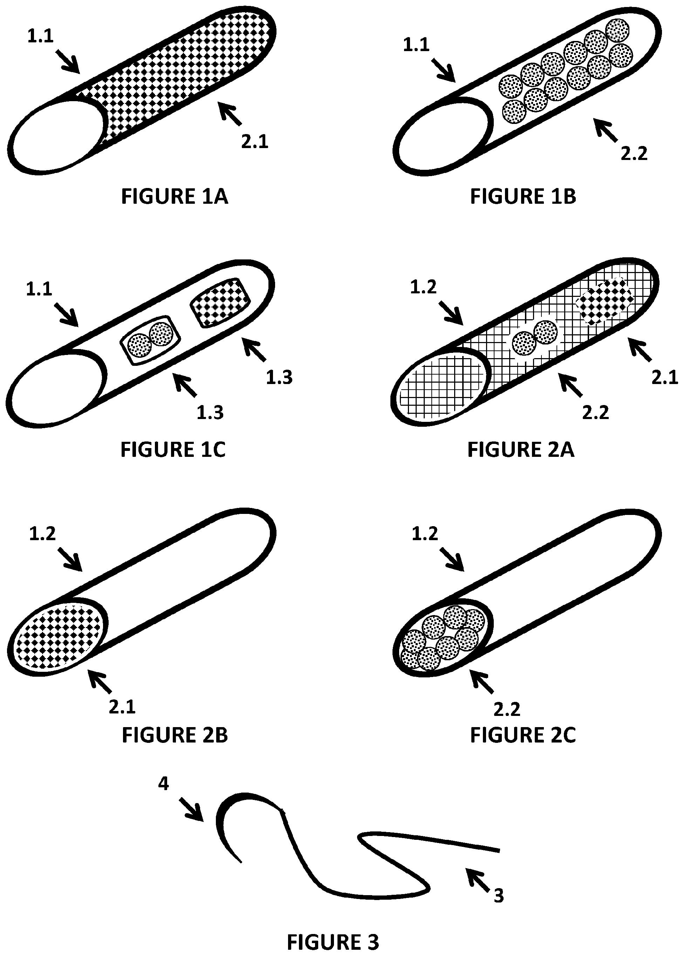

**Figure 1** is a schematic representation of three embodiments of the sutures provided by the present invention. In these embodiments, the suture is an elongated thread **1.1,** coated by a dermal filler, which according to some embodiments is formulated as a continuous layer or phase **2.1** (Fig. 1A) or as a plurality of particles **2.2** (Fig. 1B). According to some embodiments the dermal filler is contained in one or more grooves **1.3** along the elongated thread **1.1,** as illustrated in Figure 1C.
**Figure 2** is a schematic representation of three embodiments of the sutures provided by the present invention. In one embodiments, the suture is an elongated porous thread having an internal cavity **1.2,** filled with a dermal filler, which according to some embodiments is formulated as a continuous layer or phase **2.1** and/or as a plurality of particles **2.2** (Fig. 2A). In other embodiments, the suture is an elongated thread having an internal cavity **1.2,** filled with a dermal filler, which according to some embodiments is formulated as a continuous layer or phase 2.1 (Fig. 2B) or as a plurality of particles **2.2** (Fig. 2C).
**Figure 3** is a schematic representation of a suture provided by the present invention **3** linked to a needle **4.**

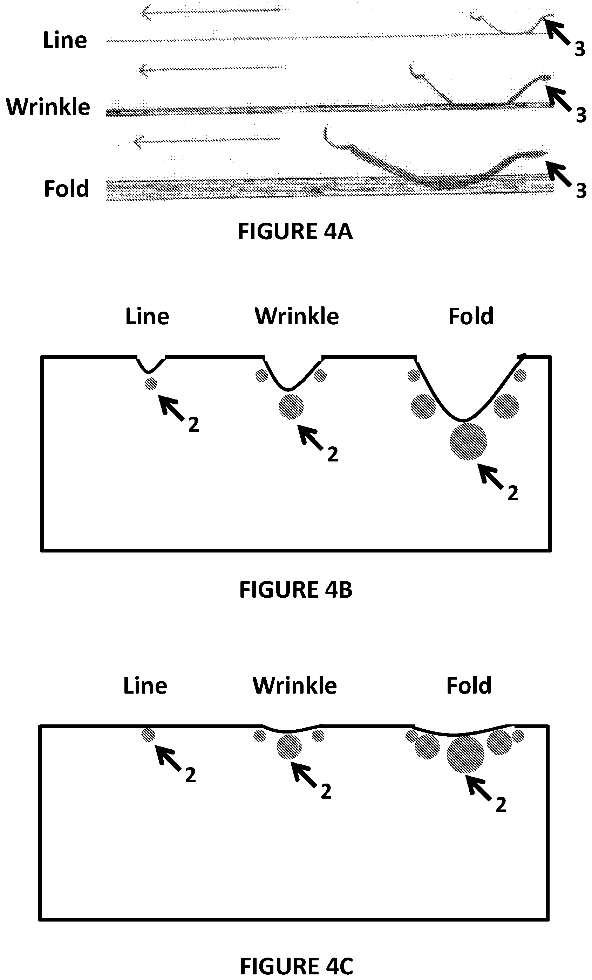

**Figure 4** is a schematic representation of a skin having a line, a wrinkle and a fold, as generally defined herein being treated by a method of the present disclosure. Figure 4B is a cross-sectional view of the skin of Figure 4A after being stitched by a suture provided by the present invention. Figure 4C is a cross-sectional view of the skin of Figure 4B, several hours, days or weeks after being stitched by a suture provided by the present invention. Different amounts of fillers **2** are represented as gray spheres of different sizes.

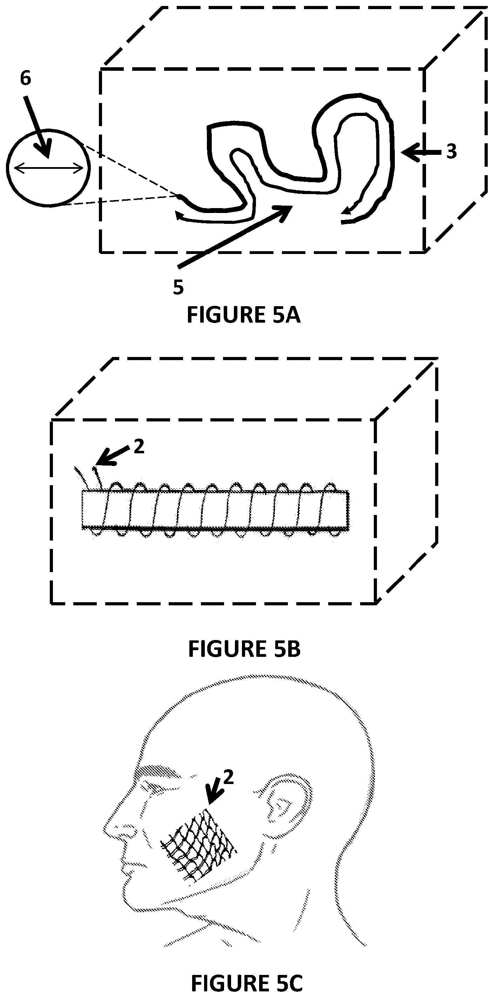

**Figure 5** is a schematic three-dimensional representation of a suture provided by the present invention, having a longitude **5** and a diameter **6** which are perpendicular to each other (Fig. 5A), of an coil-like filamentous structure of a filler **2** obtained by the sutures of the present invention (Fig. 5B), and of mesh of a filler **2** obtained by the sutures of the present invention formed in or implanted to the cheek of a human subject (Fig. 5C).

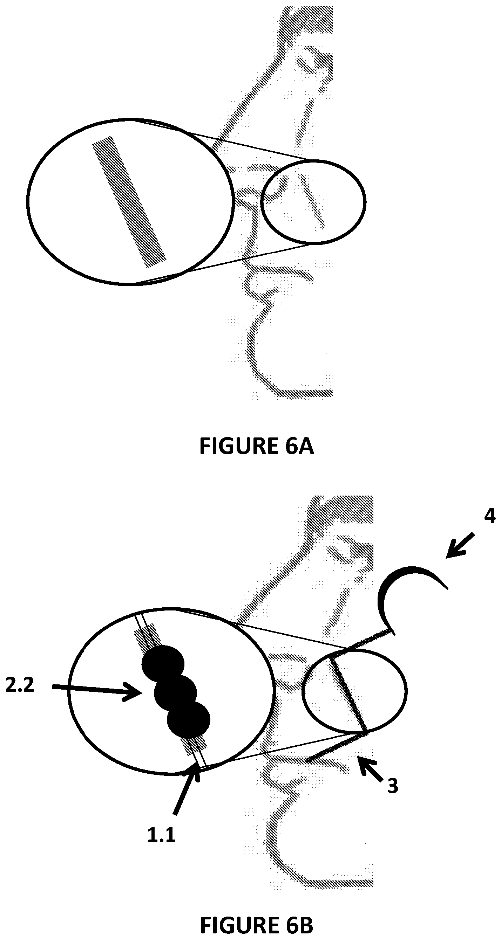

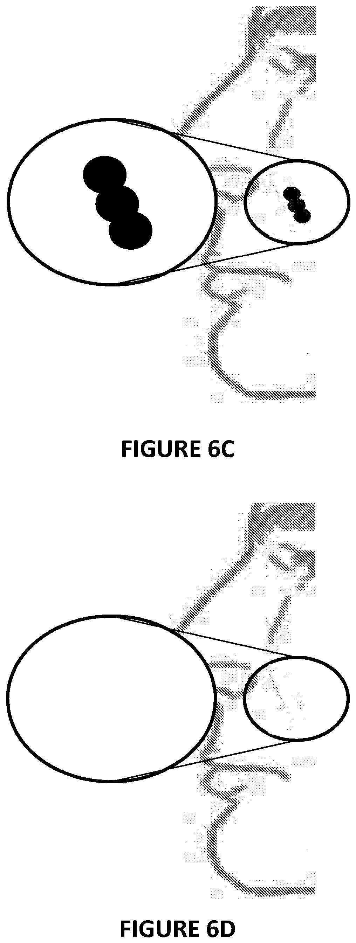

**Figure 6** is a schematic representation of an absorbable suture provided by the present disclosure used in a method suture provided by the present disclosure. Figure 6A shows a magnification of a line (wrinkle) in the nasolabial skin of a subject. Figure 6B shows the line after being stitched along its length by an absorbable suture **3** provided by the present disclosure connected to a needle **4.** In the expanded view, it can be seen that the absorbable suture **3** is made from an elongated thread **1.1** coated by a plurality of dermal filler particles **2.2.** Figure 6C shows the area of the line beneath the skin after the absorbable suture **3** provided by the present disclosure was absorbed. In the expanded view, it can be seen that the absorbable suture **3** was absorbed, while the plurality of dermal filler particles **2.2** remain in the skin in the area of the line. Figure 6D shows the surface of the area of the line after treatment. In the expanded view, it can be seen that the line has vanished.

### DETAILED DESCRIPTION OF THE INVENTION

The present invention relates to solid sutures, in particular, surgical sutures, which are used in stitching of human tissues, particularly the skin, which advantageously comprise materials used for skin augmentation, known as dermal or skin fillers. More specifically, the solid sutures of the present invention are biocompatible, meaning they do not invoke significant immunogenic response when transplanted in humans. These sutures are "biodegradable" according to some embodiments, meaning they are degraded over time when transplanted in humans, and/or according to some embodiments "bioabsorbable", meaning the products of suture degradation are naturally absorbed in the tissue. The combination of biocompatible solid matrixes and dermal fillers provides healthcare professionals, such as plastic surgeons, with new tools for achieving improved cosmetic effects, which up till now were reached by elaborate, cumbersome surgical techniques, or were simply unattainable due to technical limitations.

According to the principles of the invention, the sutures of the present invention advantageously allow healthcare professionals, such as plastic surgeons, to administer predetermined dosages of practically any dermal fillers in unprecedented accuracy and resolution in substantially any spatial configuration, limited only by a surgeons' proficiency. No technique disclosed thus far is capable of administering and spreading skin augmenting fillers in a highly homogenous fashion across skin lines as the sutures and techniques provided by the present invention. In addition, no technique disclosed thus far relates to or is capable of administering and uniformly distributing rigid, inflexible skin augmenting fillers to the skin of patients without resorting to repeated injections of such fillers.

The present invention provides, in an aspect, a surgical suture, comprising (i) at least one flexible solid matrix formulated in the shape of a thread, the thread having an ultimate tensile strength sufficient to pull the thread through a human skin or sub-cutis and manipulate the thread once in the skin or sub-cutis such that the integrity of the thread is not substantially compromised; the at least one flexible solid matrix being in association with (ii) at least one dermal filler, wherein the dermal filler is chemically different from the flexible solid matrix, and wherein the dermal filler gradually dissociates from the flexible solid matrix while in the skin or the sub-cutis.

According to some embodiments, the term "skin augmentation" refers to increasing the volume of the treated skin and/or sub-cutis. According to some embodiments, the term "skin augmentation" refers to increasing the apparent volume of the treated skin. The term "surgical suture" as used herein refers to any suture approved for use in cosmetic and/or therapeutic surgery. As used herein, the terms "suture", "the suture of the invention" "augmentation suture", "a soft tissue augmentation suture" and "skin augmentation suture" are used interchangeably and refer to a suture comprising at least one biocompatible skin augmentation material. It is to be understood that a skin augmentation suture according to the present invention is suitable for filling of skin and/or the sub-cutis. The term "flexible" as used herein refers to the ability of the solid matrix and/or the surgical suture to be elastically deformed as required during surgery or suturing. The term "thread" is used herein in a very broad sense to include any single or multiple strand, fiber or yarn, either mono-filament or multi-filament. The term "ultimate tensile strength" as used herein generally refers to the capacity of a material or structure to withstand loads tending to elongate. The phrase "the integrity of the thread is not substantially compromised" as used herein generally means that the thread keeps its structural integrity during being pulled and manipulated in the skin or sub-cutis of a human, e.g. is not substantially torn, broken, fails, deforms or loses its ultimate tensile strength. The term "in association with" as used herein refers to a direct or indirect physical contact or chemical attachment between the dermal filler and the solid matrix. As used herein, the terms "filler" and "dermal filler" refer to agents and compositions useful for augmentation of skin, e.g. to correct or minimize skin defects. A filler material generally fulfils a structural function. It is configured to fill in a defect, hole, space or cavity in a tissue and/or provide an environment in which host cells adhere to the filler material and grow and produce other factors (e.g., chemotactic factors) resulting from the growth of new tissue. In certain embodiments, the gap-filling function of the filler is temporary and only lasts until the host cells migrate into the area and form new tissue. Dermal and sub-dermal fillers are materials to be introduced to the body, usually to the skin, in order to correct wrinkles, expression lines, lipodistrophy and other traits that an individual may want or may need to correct, mainly in the face. These compounds are used to fill the cavities or grooves created in the skin tissue due to different causes. Among the most used fillers worldwide are acrylamides (polyacrylamide and polyalkylimide compounds), hyaluronic acid compounds, polylactic acid, polymethylmethacrylate compounds, calcium hydroxyapatite, collagen, polytetrafluoroethylene and silicone oil.

Soft Tissue Fillers, also known as injectable implants, dermal fillers, or wrinkle fillers further include materials approved by the Food and Drug Administration (FDA), European Medicines Agency (EMEA) or any corresponding agency for use in helping to create a smoother and/or fuller appearance in the face, including nasolabial folds, cheeks and lips and for increasing the volume of the back of the hand. Most soft tissue fillers have a temporary effect, because they contain materials that are absorbed by the body over time. In certain embodiments, the materials used in soft tissue fillers are selected from the group consisting of absorbable (temporary) materials and non-absorbable (permanent) materials. In certain embodiments, the absorbable materials are selected from the group consisting of collagen, hyaluronic acid, calcium hydroxyapatite and poly-L-lactic acid (PLLA). Each possibility represents a separate embodiment of the invention. In certain embodiments, the non-absorbable material is polymethylmethacrylate beads.

The term "chemically different" as used herein refers to the different material(s) or composition(s) of the solid matrix and the dermal filler. In certain embodiments, the chemical difference is selected from the group consisting of size, shape, formulation, chemical structure, chemical make-up, rigidity, flexibility and any combination thereof. This term specifically excludes any surgical sutures consisting of solid formulation(s) of dermal filler materials, such as hyaluronic acid. The phrase "gradual dissociation while in the skin or the sub-cutis" refers to the effect of the conditions found in the skin or in the sub-cutis, such as pH, water content and/or temperature, on the physical separation between the dermal filler and the solid matrix.

In certain embodiments, the dermal filler cannot be formulated to withstand the forces or pressures involved in penetrating the skin surface, moving through the skin, moving below the skin, or stitching the skin. Each embodiment represents a separate embodiment of the invention.

In certain embodiments, the dermal filler cannot be formulated as a thread capable of withstanding the forces or pressures involved in penetrating the skin surface, moving through the skin, moving below the skin, or stitching the skin. Each embodiment represents a separate embodiment of the invention.

In certain embodiments, the dermal filler is biocompatible. In certain embodiments, the dermal filler is absorbed by the body or the skin into which it is administered within 1 to 2 years. In certain embodiments, the dermal filler promotes collagen formation within the body or the skin into which it is administered. In certain embodiments, the dermal filler have minimal side-effects within the body or the skin into which it is administered. In certain embodiments, the dermal filler does not produce granules or lumps within the body or the skin into which it is administered. In certain embodiments, the dermal filler is substantially inert. In certain embodiments, the dermal filler does not chemically interact with other compounds co-administered to the body or skin. In certain embodiments, the dermal filler is produced in variable sizes, shapes or forms. In certain embodiments, the size, shape or form of the dermal filler is predetermined. In certain embodiments, the size, shape or form of the dermal filler is tailored according to the depth, volume or visibility of an undesired line, area, wrinkle, depressed scar or fold in the body or skin of a subject.

In certain embodiments, the solid matrix is configured to carry and serve as a vehicle to the dermal filler in many configurations or formulations. In certain embodiments, at least one section of the thread is formulated in the shape of (i) a full thread, and is at least partly externally coated by the dermal filler; (ii) a grooved thread, and the groove is at least partly filled with the dermal filler; (iii) a porous thread, and is at least partly filled with the dermal filler; or (iv) a hollow thread, and is at least partly filled with the dermal filler. Each possibility represents a separate embodiment of the invention.

In certain embodiments, the dermal filler is formulated in the shape of a continuous phase. The term "continuous phase" as used herein refers to a segment, sheet, layer or phase which is not interrupted by any other material. In certain embodiments, the dermal filler and an agent which promotes the dissociation of the dermal filler from the thread while in the skin or the sub-cutis are formulated together in the shape of a continuous phase. In certain embodiments, the dermal filler and an agent which promotes the dissociation of the dermal filler from the thread while in the skin or the sub-cutis are formulated in the shape of separate continuous phases. In certain embodiments, the dermal filler and an agent which promotes the dissociation of the dermal filler from the thread while in the skin or the sub-cutis are formulated together in the shape of a plurality of particles.

The distribution of the dermal filler over, as part of, or within the solid matrix comprises many configurations. In certain embodiments, the dermal filler is substantially evenly and/or homogenously distributed along at least one section of the thread. Each possibility represents a separate embodiment of the invention. The term "evenly distributed" as used herein means that the dermal filler forms a substantially homogenous sheet or phase when in contact with the biodegradable solid matrix. In certain embodiments, the dermal filler is in different shapes evenly distributed as a mixture along the thread.

In certain embodiments, the thread is at least partly coated by the dermal filler. The phrase "at least partly coated" as used herein refers to coating of at least 10%, at least 20%, at least 30%, at least 40%, at least 50%, at least 60%, at least 70%, at least 80%, or at least 90% of the external or the internal surface of the thread by the dermal filler. Each possibility represents a separate embodiment of the present invention. In certain embodiments, the thread is substantially fully coated by the dermal filler.

In certain embodiments, the thread is at least partly filled by the dermal filler. The phrase "at least partly filled" as used herein refers to a volume of the dermal filler filling at least 10%, at least 20%, at least 30%, at least 40%, at least 50%, at least 60%, at least 70%, at least 80%, or at least 90% of the volume of the internal cavity of the thread by the dermal filler. Each possibility represents a separate embodiment of the present invention. In certain embodiments, the internal cavity of the thread is substantially fully filled by the dermal filler.

In certain embodiments, the average diameter of the thread is 180 to 4600 microns. In certain embodiments, the average diameter of the thread is 180 to 1500 microns. The term "average diameter" as used herein refers to the longest axis in a cross-section of the thread or the particle, whichever is relevant. In embodiments where the cross-section of the thread is substantially a circle, the average diameter is the diameter of the circle. Similarly, in embodiments where the cross-section of the thread is substantially a rectangle, the average diameter is the distance between two opposing corners of the rectangle. It should be understood that a cross-section of the thread comprises any shape, such as a triangle, a polygonal, an elliptic shape, etc..

In certain embodiments, the average diameter of the thread is 20 to 5000 microns. In certain embodiments, the average diameter of the thread is 20 to 800 microns. In certain embodiments, the average diameter of the thread is 50 to 400 microns. In certain embodiments, the average diameter of the thread is 100 to 400 microns. In certain embodiments, the average diameter of the thread is 70 to 350 microns. In certain embodiments, the average diameter of the thread is 100 to 300 microns. In certain embodiments, the average diameter of the thread is 150 to 200 microns. In certain embodiments, the average diameter of the thread is 100 microns. In certain embodiments, the average diameter of the thread is 150 microns. In certain embodiments, the average diameter of the thread is 200 microns. In certain embodiments, the average diameter of the thread is 20 to 150 microns. In certain embodiments, the average diameter of the thread is 150 to 800 microns. The term "micron" refers to micrometer, the millionth part of a meter.

In certain embodiments, the thread is at least 1 cm, at least 10 cm, or at least 100 cm in length. Each possibility represents a separate embodiment of the invention.

The term "biodegradable solid matrix" refers to a solid matrix that is capable of being completely or substantially degraded, eroded or absorbed when exposed to either an in-vivo environment or an in-vitro environment having physical, chemical, or biological characteristics substantially similar to those of the in vivo-environment within a living organism. A material is capable of being degraded or eroded when it can be gradually broken-down, resorbed, absorbed and/or eliminated by, including, hydrolysis, enzymolysis, oxidation, metabolic processes, bulk or surface erosion, and the like within a living organism. The term "living organism" as used herein refers to living human and animals. The biodegradable material degrades into non-toxic components in a living organism, and its degradation may not cause substantial tissue irritation or necrosis at the target tissue site. As used herein, the term "biodegradable" refers to a material which is naturally degraded when in a subject's body, by e.g. enzymatic activity, chemical dissolution or otherwise. As used herein, the term "biocompatible" refers to a material which does not elicit significant undesirable and/or toxic local or systemic effects when administered to a subject. In certain embodiments, the thread is biodegradable and/or absorbable. In certain embodiments, the thread comprises a material selected from the group consisting of glycolide, polyglycolide, glycolic acid, polyglycolic acid, lactide, L-lactide, lactic acid, poly(lactic-co-glycolic acid) (PLGA), glycolide/lactide copolymer, caprolactone, glycolide/epsilon- caprolactone copolymer, dioxanone, polydioxanone, trimethyl carbonate, ethyl glycol, salts thereof, esters thereof, hydrates thereof, and any combination thereof. Each possibility represents a separate embodiment of the invention. In certain embodiments, the biodegradable solid matrix is of a biological origin. In certain embodiments, the biodegradable solid matrix is made of bovine or sheep intestine.

In certain embodiments, the thread is substantially degraded in 1-30 weeks, 1-20 weeks, 1-10 weeks, 1-30 days, 1-20 days, or 1-10 days under physiological conditions. Each possibility represents a separate embodiment of the invention.

In certain embodiments, the thread is non-biodegradable or non-bioabsorbable. In certain embodiments, the thread comprises a material selected from the group consisting of nylon, silk, polypropylene, polyester, stainless steel, and any combination thereof. Each possibility represents a separate embodiment of the invention. In certain embodiments, wherein the thread is non-biodegradable or non-bioabsorbable, the dermal filler is contained in grooves, holes or tunnel in the suture. In certain embodiments, the suture is removed after days to weeks after the skin augmenting filler moved into the skin or sub-cutis by osmosis or diffusion. In certain embodiments, the dermal filler is mixed with Poly-Etylene Glycol (PEG), Poly Etylene Oxide (PEO), magnesium sulphate, magnesium citrate, or a combination thereof.

Dermal fillers, also termed "skin augmentation materials", which are comprised in the composition of the invention, are effective dermal fillers, if possible, approved by the U.S. Food and Drug administration and/or the matching organization of Europe (CHAMP). Other skin augmentation materials that are not yet approved for human use may also be used in the future. Skin augmentation materials that are already approved include, but not limited to, fillers comprising structural proteins, polysaccharides or synthetic polymers. In some embodiments skin augmentation materials include hydroxyapatite, collagen, such as reconstituted bovine collagen products including, but not limited to, ZYDERM I^{®}, ZYDERM II^{®} and ZYPLAST^{®} (Collagen Corporation); natural human collagen COSMODERM^{™} and COSMOPLAST^{™} (INAMED); and endogenous collagen from the subject, AUTOLOGEN^{®} produced by Collagenesis. In some embodiments, additional dermal fillers are selected from those comprising hyaluronic acid, including but not limited to, such products as HYLAFORM^{®} gel manufactured by INAMED and Genzyme Corporations, derived from the rooster combs of domestic fowl; and RESTYLANE^{®} manufactured by Medicis, a hyaluronic acid derivative derived from streptococcal bacterial fermentation. Hyaluronic acid according to the present invention includes both non-cross-liked and/or cross-linked hyaluronic acid derivatives as are well known in the art. Hyaluronic acid and its derivatives are: solid, semi-solid or liquid. Each possibility represents a separate embodiment of the present invention. According to some embodiments, collagen according to the invention is selected from the group consisting of: allogeneic collagen, xenogeneic collagen and a combination thereof. Each possibility represents a separate embodiment of the present invention. According to other embodiments, a skin augmentation material is human cadaveric dermis cultivated from a cadaver, such as, but not limited to, the materials having the brand names Cymetra, Dermalogen, Alloderm and Fascian.

According to some embodiments, a biodegradable natural filler is selected from the group consisting of: bovine collagen, porcine collagen, recombinant collagen, human collagen, gelatin, hyaluronic acid, hyaluronic acid derivative, hydroxyapatite, dried acellular particulate dermal matrix, allogeneic fat and combinations thereof. Each possibility represents a separate embodiment of the present invention. According to some embodiments, a biodegradable synthetic polymer is selected from the group consisting of: poly-L-lactic acid, polyethylene oxide, carboxymethylcellulose and combinations thereof. Each possibility represents a separate embodiment of the present invention. According to some embodiments, a non-biodegradable synthetic polymer is selected from the group consisting of: polymethyl methacrylate, polymethyl methacrylate beads, silicones, silicone rubber, expanded polytetrafluoroethylene, polyacrylamide, polyalkylimide and combinations thereof. Each possibility represents a separate embodiment of the present invention.

In certain embodiments, the dermal filler is formulated as a solid. In certain embodiments, the dermal filler is formulated as a semi-solid. In certain embodiments, the dermal filler is formulated as a liquid.

While the field of materials used as dermal fillers intended to augment skin appearance is continually growing, a physical and technical challenge still remains in administering solid, rigid and inflexible dermal fillers to the skin. Although surgical sutures are configured to withstand the forces applied during surgery, this is not the case for rigid dermal fillers. Therefore, up to the present invention, the administration of rigid dermal fillers has been limited techniques such as repeated, multiple bolus injections of liquid compositions of these fillers along the treated skin defect. These techniques, however, involve tedious labor and increased injection-site adverse effects, and often result in a non-attractive, non-homogenous "chain-of-pearls" look of the skin. All of these deficiencies are obviated by the present invention.

In certain embodiments, the dermal filler comprises at least one liquid dermal filler. In certain embodiments, the dermal filler comprises at least one semi-solid dermal filler. In certain embodiments, the dermal filler is formulated as a liquid. In certain embodiments, the dermal filler is formulated as a semi-solid. In certain embodiments, the dermal filler comprises at least one rigid dermal filler. In certain embodiments, the dermal filler is formulated as a solid. The terms "liquid", "semi-solid" and "solid" generally refer to the states of matter. In certain embodiments, the terms "liquid", "semi-solid" and "solid" refer to states of matter at room temperature. In certain embodiments, the terms "liquid", "semi-solid" and "solid" refer to states of matter at 10°C to 25°C. The terms "liquid", "semi-solid" and "solid" refer to the states of matter at the temperature of the human adult body. The terms "liquid", "semi-solid" and "solid" refer to the states of matter at 36°C to 37°C.

The term "rigid dermal filler" as used herein refers to any dermal filler which would suffer any kind of a permanent structural insult under forces applied during surgery, including the forces applied during plastic surgery to reduce the depth, volume or visibility of an undesired line, area, wrinkle, depressed scar or fold in the skin of a subject. In certain embodiments, the rigid dermal filler is not flexible. In certain embodiments, the rigid dermal filler cannot be elastically deformed as required during surgery or suturing.

In certain embodiments, the rigid dermal filler has (i) an ultimate tensile strength of up to 300 MPa; (ii) a compressive strength of up to 1000 MPa; (iii) an elastic modulus of at least 80 GPa; (iv) a fracture toughness of up to 1 MPa.m-1/2; or (v) any combination of (i) to (iv). Each possibility represents a separate embodiment of the invention.

In certain embodiments, the rigid dermal filler has (i) an ultimate tensile strength of up to 300 MPa; (ii) a compressive strength of up to 1000 MPa; (iii) an elastic modulus of at least 80 GPa; (iv) a fracture toughness of up to 1 MPa.m-1/2; or (v) any combination of (i) to (iv); when the rigid dermal filler is in the shape of a full cylinder of 5 mm or more in length and a diameter of 500 microns. Each possibility represents a separate embodiment of the invention.

In certain embodiments, the rigid dermal filler has (i) an ultimate tensile strength of 40 to 300 MPa; (ii) a compressive strength of 500 to 1000 MPa; (iii) an elastic modulus of 80 to 120 GPa; (iv) a fracture toughness of 0.6 to 1 MPa.m-1/2; or (v) any combination of (i) to (iv). Each possibility represents a separate embodiment of the invention.

In certain embodiments, the rigid dermal filler has (i) an ultimate tensile strength of 40 to 300 MPa; (ii) a compressive strength of 500 to 1000 MPa; (iii) an elastic modulus of 80 to 120 GPa; (iv) a fracture toughness of 0.6 to 1 MPa.m-1/2; or (v) any combination of (i) to (iv); when the rigid dermal filler is in the shape of a full cylinder of 5 mm or more in length and a diameter of 500 microns. Each possibility represents a separate embodiment of the invention.

Minerals are a naturally occurring chemical compound, usually of crystalline form, some of which are used as rigid dermal filler. Mineraloids are mineral-like substances that do not demonstrate crystallinity, some of which are used as rigid dermal filler. In certain embodiments, the rigid dermal filler is a ceramic, a mineral or a mineraloid. Minerals are described as brittle, ductile, malleable, sectile, flexible, or elastic. In certain embodiments, the rigid dermal filler is brittle, malleable, ductile or sectile. In certain embodiments, the rigid dermal filler is brittle.

The most common scale of mineral hardness measurement is the ordinal Mohs hardness scale, ranges from talc to diamond. In certain embodiments, the rigid dermal filler has Mohs hardness score of 2 to 10. In certain embodiments, the rigid dermal filler has Mohs hardness score of 5 to 10.

In certain embodiments, the rigid dermal filler is formulated in the shape of a plurality of particles. In certain embodiments, the average diameter of the particles in the plurality of particles is 500 microns at most. In certain embodiments, the average diameter of the particles in the plurality of particles is 100 microns at most. In certain embodiments, the average diameter of the particles in the plurality of particles is 5 to 500 microns. In certain embodiments, the average diameter of the particles in the plurality of particles is 10 to 100 microns. In certain embodiments, the average diameter of the plurality of particles is 10 to 50 microns. In certain embodiments, the average diameter of the plurality of particles is 20 to 50 microns. In certain embodiments, the average diameter of the plurality of particles is 25 to 45 microns. The term "particle" herewith is referred as a spherical particle, a rod-like particle, a filament-like particle, a disk-like particle, a polygonal particle or a combination thereof. In certain embodiments, the particle is a nanoparticle, a microparticle, a microparticle or a combination thereof. Each possibility represents a separate embodiment of the invention. As used herein, the terms "a plurality of" and "a multiplicity of" are used interchangeably and refer to at least two. As used herein, the terms "made of" and "composed of" are used interchangeably.

In certain embodiments, the dermal filler is selected from the group consisting of an apatite, hyaluronic acid, polycaprolactone, polymethylmethacrylate, silicone, polylactic acid, collagen, gelatin, dermal graft, processed dermal graft, dried acellular particulate dermal matrix, fat cells, stem cells, chondrocytes, salts thereof, esters thereof, hydrates thereof, derivatives thereof, solvates thereof, and any combination thereof. Each possibility represents a separate embodiment of the invention.

In certain embodiments, the dermal filler comprises an apatite, a salt thereof, an ester thereof, a hydrate thereof, a derivative thereof, a solvate thereof, and any combination thereof. Apatite is a group of phosphate minerals, usually referring to hydroxyapatite, fluorapatite and chlorapatite, with high concentrations of OH⁻, F⁻ and Cl⁻ ions, respectively, in the crystal. The formula of the admixture of the four most common endmembers is written as Ca₁₀(PO₄)₆(OH,F,Cl)₂. In certain embodiments, the term "apatite" refers to hydroxyapatite, fluorapatite and/or chlorapatite, in pure form or in any combination thereof. In certain embodiments, apatite, hydroxyapatite, fluorapatite and chlorapatite are derivatives of each other. In certain embodiments, the dermal filler comprises hydroxyapatite, a salt thereof, an ester thereof, a hydrate thereof, a derivative thereof, a solvate thereof, and any combination thereof.

In certain embodiments, the thread is biodegradable or bioabsorbable, and wherein the hydroxyapatite dermal filler is formulated in the shape of a plurality of particles having an average diameter of 10 to 100 microns evenly distributed along at least one section of the thread.

In certain embodiments, the surgical suture described above further comprises an agent which promotes the dissociation of the dermal filler from the thread while in the skin or the sub-cutis. In certain embodiments, the agent is a hygroscopic, diffusible or osmotic agent.

In certain embodiments, the surgical suture described above further comprises a needle. In certain embodiments, the diameter of the needle is smaller, the same or larger compared to the diameter of the surgical suture. In certain embodiments, the needle is selected from the group consisting of a straight needle, a 1/4 circle needle, a 3/8 circle needle, a 1/2 circle needle, a 5/8 circle needle, a compound curve needle, a half curved (also known as ski) needle, and a half curved at both ends of a straight segment (also known as canoe) needle. Needles are classified by their point geometry; including: taper (needle body is round and tapers smoothly to a point); cutting (needle body is triangular and has a sharpened cutting edge on the inside curve); reverse cutting (cutting edge on the outside); prime cutting needle; trocar point or tapercut (needle body is round and tapered, but ends in a small triangular cutting point); blunt points for sewing friable tissues; side cutting or spatula points (flat on top and bottom with a cutting edge along the front to one side) for eye surgery. Each possibility represents a separate embodiment of the invention. In certain embodiments, the needle is rigid enough to penetrate a skin. In certain embodiments, the needle is made of ceramic, metal or plastic.

In certain embodiments, the surgical suture described above further comprises a cosmetic agent or a biologically-active agent. The term "biologically-active agent" as used herein refers to any agent inflicting a biological effect when introduced into a subject's body. The term "cosmetic agent" as used herein refers to any agent inflicting a cosmetic effect when introduced into a subject's body.

In certain embodiments, the cosmetic agent is a pigment. In certain embodiments, the biologically-active agent is selected from the group consisting of an enzyme, a drug, and a combination thereof. In certain embodiments, the enzyme is collagenase or elastase. In certain embodiments, the drug is an antibiotic agent, retinoic acid, a glycosaminoglycan (GAG), a botulinum toxin, an analgesic, an anti-scarring drug, or any combination thereof. In certain embodiments, the anti-scarring drug is a steroid.

Described but not part of the invention is a kit, comprising at least one surgical suture according to the present invention as described above.

Described but not part of the invention is a kit, comprising a plurality of different surgical sutures as described above.

The present invention further provides, in another aspect, a surgical suture according to the present disclosure as described above, for use in a cosmetic or therapeutic method of filling an undesired line, area, wrinkle, depressed scar or fold in skin.

In certain embodiments, the method is for reducing the depth, volume or visibility of an undesired line, area, wrinkle, depressed scar or fold in the skin of the subject. In certain embodiments, the surgical suture is to be inserted into the epidermis, the dermis, the hypodermis or the sub-cutis of the subject. In certain embodiments, the surgical suture is inserted at least 0.01 mm into the skin. In certain embodiments, the surgical suture is inserted up to 5 mm into the skin or in the sub-cutis. In certain embodiments, the surgical suture is inserted 0.01 mm to 5 mm into the skin or in the sub-cutis. In certain embodiments, the surgical suture is inserted 1 mm to 2 mm into the skin or in the sub-cutis. In certain embodiments, the surgical suture is inserted into the epidermis. In certain embodiments, the surgical suture is inserted into the dermis. In certain embodiments, the surgical suture is inserted into the sub-cutis. In certain embodiments, the surgical suture is inserted below the epidermis. In certain embodiments, the surgical suture is inserted below the dermis. In certain embodiments, the surgical suture is inserted below the sub-cutis. Each possibility represents a separate embodiment of the disclosure.

The present disclosure further provides, in another aspect, a cosmetic or therapeutic method of reducing the depth, volume or visibility of an undesired line, area, wrinkle, depressed scar or fold in the skin of a subject in need thereof, comprising the steps of (i) obtaining a surgical suture according to the present disclosure as described above; (ii) optionally, connecting a needle to the surgical suture of (i); (iii) optionally, performing local anesthesia in the undesired line, area, wrinkle, depressed scar or fold in the skin of the subject; and (iv) stitching the undesired line, area, wrinkle, depressed scar or fold in the skin of the subject with the surgical suture of (i) or (ii).

In certain embodiments, the method results in reducing the depth, volume or visibility of an undesired line, wrinkle, depressed scar or fold in the skin of the subject. In certain embodiments, the stitching step is performed along the undesired line, area, wrinkle, depressed scar or fold. In certain embodiments, the stitching step is repeated at least once. In certain embodiments, the stitching step is repeated until the undesired line, area, wrinkle, depressed scar or fold is substantially ameliorated or no longer visible. In certain embodiments, the surgical suture comprises at least a segment composed of a non-biodegradable or non-bioabsorbable thread, and the method further comprises the step of removing the segment from the body of the subject. In certain embodiments, the step of removing the segment from the body of the subject is affected few hours to few weeks after the stitching. In certain embodiments, the diameter of the suture is predetermined according to the size, depth or volume of the line, area, wrinkle, depressed scar or fold to be filled. In certain embodiments, the content of the dermal filler in the suture is predetermined according to the size, depth or volume of the line, area, wrinkle, depressed scar or fold to be filled.

In certain embodiments, the surgical suture is inserted into the epidermis, the dermis or the sub-cutis of the subject. In certain embodiments, the surgical suture is inserted at least 0.01 mm into the skin. In certain embodiments, the surgical suture is inserted up to 5 mm into the skin or sub-cutis. In certain embodiments, the surgical suture is inserted 0.01 mm to 5 mm into the skin or sub-cutis. In certain embodiments, the surgical suture is inserted 1 mm to 2 mm into the skin or sub-cutis. In certain embodiments, the surgical suture is inserted into the epidermis. In certain embodiments, the surgical suture is inserted into the dermis. In certain embodiments, the surgical suture is inserted into the sub-cutis. In certain embodiments, the surgical suture is inserted below the epidermis. In certain embodiments, the surgical suture is inserted below the dermis. In certain embodiments, the surgical suture is inserted below the sub-cutis. Each possibility represents a separate embodiment of the disclosure.

The present disclosure further provides, in another aspect, a cosmetic or therapeutic method of forming a filamentous structure of at least one dermal filler in a body of a subject in need thereof, comprising the steps of (i) obtaining a surgical suture according to the present disclosure as described above; (ii) optionally, connecting a needle to the surgical suture of (i); and (iii) placing the surgical suture of (i) or (ii) within the body of the subject as to form the filamentous structure within the body of the subject.

In certain embodiments, the average diameter of the filamentous structure is less than 1 mm. In certain embodiments, the average diameter of the filamentous structure is less than 500 micron. In certain embodiments, the average diameter of the filamentous structure is less than 250 micron. In certain embodiments, the average diameter of the filamentous structure is less than 200 micron. In certain embodiments, the average diameter of the filamentous structure is 20 to 5000 microns. In certain embodiments, the average diameter of the filamentous structure is 50 to 400 microns. In certain embodiments, the average diameter of the filamentous structure is 70 to 350 microns. In certain embodiments, the average diameter of the filamentous structure is 100 to 300 microns. In certain embodiments, the average diameter of the filamentous structure is 150 to 200 microns. In certain embodiments, the average diameter of the filamentous structure is 100 microns. In certain embodiments, the average diameter of the filamentous structure is 150 microns. In certain embodiments, the average diameter of the filamentous structure is 200 microns. In certain embodiments, the average diameter of the filamentous structure is 20 to 150 microns. In certain embodiments, the average diameter of the filamentous structure is 150 to 800 microns. The term "filamentous" is used herein in its most general sense, and generally refers to elongated structures. The term "filamentous" further generally refers to and includes structures having a first axis which is at least 2, at least 10, at least 100, at least 100, or at least 1000 larger than a second axis which is perpendicular to the first inner axis, e.g. its length is at least two times larger than its diameter.

In certain embodiments, the average diameter of the filamentous structure throughout its length is 0.1 to 10 mm. In certain embodiments, the average diameter of the filamentous structure throughout its length is 1 to 5 mm.

According to the principles of the present invention, the sutures of the invention, or the fillers inserted into the skin by the sutures of the invention, form substantially two-dimensional (2D) and/or three-dimensional (3D) spatial structures within the skin. The structures are of all shapes, wherein the contour of these shapes is made of the sutures of the invention or the fillers inserted into the skin by the sutures of the invention.

The present disclosure further provides, in another aspect, a cosmetic or therapeutic method of introducing an implant into a body of a subject in need thereof, comprising the steps of (i) obtaining a surgical suture according to the present disclosure as described above; (ii) forming the surgical suture of (i) in the shape of the implant; (iii) optionally, connecting a needle to the implant to allow fixation of the implant under the skin or under a mucous membrane of the subject; and (iv) placing the implant within the body of the subject as needed.

According to some embodiments, the suture comprises a biodegradable carrier. According to some embodiments, the biodegradable carrier is selected from the group consisting of: a salt, a biodegradable polymer and a combination thereof. According to some embodiments, the biodegradable polymer is a polymer selected from the group consisting of: polyethylene glycol (PEG), polyethylene oxide (PEO), Polyglactin 910, Polyglecaprone 25, Polydioxanone, Lactomer 9-1, Glycomer 631, Polyglyconate and a combination thereof. According to some embodiments, the salt is selected from the group consisting of: sodium sulfate, sodium chloride, magnesium sulfate, magnesium citrate, magnesium chloride and a combination thereof. According to some embodiments, the biodegradable carrier comprises magnesium sulfate and polyethylene glycol. According to some embodiments, the suture comprises hydroxyapatite particles, magnesium sulfate, polyethylene oxide, polyethylene glycol, or a combination thereof.

In certain embodiments, the surgical sutures in the kit are different by the shape of the thread. In certain embodiments, the surgical sutures are different by the average diameter of the thread. In certain embodiments, the surgical sutures are different by material of the thread. In certain embodiments, the surgical sutures are different by the rigidity of the dermal filler. In certain embodiments, the surgical sutures are different by the dermal filler. In certain embodiments, the surgical sutures are different by the shape of the dermal filler. In certain embodiments, the surgical sutures are different by the size of the dermal filler. In certain embodiments, the surgical sutures are different by the amount of dermal filler.

While the present invention has been described with reference to certain embodiments, it will be understood by those skilled in the art that various changes may be made and equivalents may be substituted without departing from the scope of the present invention. In addition, many modifications may be made to adapt a particular situation or material to the teachings of the present invention without departing from its scope. Therefore, it is intended that the present invention not be limited to the particular embodiment disclosed, but that the present invention will include all embodiments falling within the scope of the appended claims.

The following examples are presented in order to more fully illustrate some embodiments of the invention.

### EXAMPLES

### Example 1. Filler-coated sutures

Filler-coated sutures according to the present invention (Figs. 1A-C) are produced by contacting a suture 1.1, such as Polyglactin 910 (ETHICON) made of 90% glycolide and 10% L-lactide, and a dermal filler, such as hydroxyapatite. The filler may be a liquid, semi-solid or solid prior to and during contact, and a liquid, semi-solid or solid in the finalized product. The filler in the final product may be formulated as a continues sheet, layer or phase 2.1 (Figs. 1A and 1C), or as substantially discrete particles 2.2 (Fig. 1B and 1C). In Figure 1C, the filler is contained in defined grooves on the surface of the suture **1.1.**

### Example 2. Filler-containing sutures

Filler-containing sutures according to the present invention (Figs. 2A-C) are produced by contacting a suture having an internal cavity 1.2 and a dermal filler, such as hydroxyapatite. The filler may be a liquid, semi-solid or solid prior to and during contact, and a liquid, semi-solid or solid in the finalized product. The filler in the final product may be formulated as a continues sheet, layer or phase 2.1 (Figs. 2A and 2B) or as substantially discrete particles 2.2 (Figs. 2A and 2C). In Figure 2A, the suture having an internal cavity 1.2 is porous.

### Example 3. Stitches

In order to decrease the inner volume of lines, wrinkles or folds in the skin (Fig. 4A), the sutures provided by the present invention 3 may be employed in stitching of bottom and/or of the walls of the depressions of the skin (Figs. 4A and 4B). This technique is especially important in filling subtle lines and wrinkles. Such stitches deposit adjustable amounts of fillers into the depressions, thus filling the volume of the depressions. The amount of filler 2 used (Figs. 4B, gray spheres) and the exact location for each amount is adjusted to obtain a beneficial cosmetic result (Fig. 4C).

### Example 4. 2D and 3D structures

In order to form a defined filamentous structure of a filler in a body of a subject (Fig. 5A), the sutures provided by the present invention 3, having a longitude 5 and a diameter 6 which are perpendicular to each other, may be inserted into the body (Fig. 5A). Figure 5B portraits an exemplary filamentous structure of a filler 2, a coil, obtainable by the use of the sutures and methods provided herein. Such coils may be beneficial in preventing blood vessels or ureters from expanding and/or in confining blood vessels or ureters to a desired and beneficial shape or volume. Figure 5C portraits an exemplary filamentous structure of a filler 2, a mesh, obtainable by the use of the sutures and methods provided herein. This structure can be very useful for cheek or malar bones augmentation.

Using the sutures and methods provided by the present disclosure, one is able to create lines, loops, webs, mesh, coils, knots, and any beneficial higher-order structures inside the body of a subject. None of the techniques known in the field are capable of forming such delicate and intricate 2D and 3D structures in-vivo.

### Example 5. Treatment

In order to treat lines (wrinkles) in the skin of a subject (Fig. 6A), a cosmetic surgeon can use a surgical suture provided by the present invention and/or a method provided by the present invention. As depict in Figure 6B, a line may be stitched along its length by an absorbable suture 3 connected to a needle 4. The absorbable suture 3 is made from an elongated thread 1.1 coated by a plurality of dermal filler particles 2.2, and thus allows pin-point targeting of the dermal filler particles 2.2 into the line. After the removal of the needle 4, and after the absorbable suture 3 is absorbed, the plurality of dermal filler particles 2.2 remain in the skin in the area of the line (Figure 6C), and the line substantially vanishes (Figure 6D).

## Claims

1. A surgical suture, comprising:
(i) at least one flexible solid matrix formulated in the shape of a thread, the thread having an ultimate tensile strength sufficient to pull the thread through a human skin or sub-cutis and manipulate the thread once in the skin or sub-cutis such that the integrity of the thread is not substantially compromised; the at least one flexible solid matrix being in association with:
(ii) at least one dermal filler; wherein the dermal filler is chemically different from the flexible solid matrix; and wherein the dermal filler is configured to gradually dissociate from the flexible solid matrix, while in the skin or the sub-cutis;
wherein at least one section of the thread is formulated in the shape of:
• a grooved thread, wherein the groove is at least partly filled with the dermal filler; or
• a porous thread, wherein the pores are at least partly filled with the dermal filler; or
• a hollow thread, at least partly filled with the dermal filler.

2. The surgical suture of claim 1, wherein at least one section of the thread is at least partly externally coated by the dermal filler.

3. The surgical suture of any one of claims 1 or 2, further comprising an agent which promotes the dissociation of the dermal filler from the thread, while in the skin or the sub-cutis; wherein the agent is a hygroscopic, diffusible, or osmotic agent.

4. The surgical suture of any one of claims 1 to 3, wherein the dermal filler is substantially evenly and homogenously distributed along at least one section of the thread.

5. The surgical suture of any one of claim 1 to 4, wherein at least one of the following holds true:
• the thread is biodegradable or bioabsorbable;
• the thread is non-biodegradable or non-bioabsorbable.

6. The surgical suture of any one of claims 1 to 5, wherein the dermal filler comprises at least one liquid dermal filler or at least one semi-solid dermal filler.

7. The surgical suture of claim 6, wherein the dermal filler is formulated as a liquid or is formulated as a semi-solid.

8. The surgical suture of any one of claims 1 to 7, wherein the dermal filler comprises at least one rigid dermal filler.

9. The surgical suture of claim 8, wherein the rigid dermal filler has:
(i) an ultimate tensile strength of up to 300 MPa;
(ii) a compressive strength of up to 1000 MPa;
(iii) an elastic modulus of at least 80 GPa;
(iv) a fracture toughness of up to 1 MPa.m ^{1/2}; or
(v) any combination of (i) to (iv).

10. The surgical suture of claim 8 or claim 9, wherein the rigid dermal filler is a ceramic, a mineral or a mineraloid.

11. The surgical suture of any one of claims 1 to 10, wherein the rigid dermal filler is formulated in the shape of a plurality of particles; wherein the average diameter of the particles in the plurality of particles is 5 to 500 microns.

12. The surgical suture of any one of claims 1 to 11, wherein the dermal filler is selected from the group consisting of an apatite, hyaluronic acid, polycaprolactone, polymethylmethacrylate, silicone, polylactic acid, collagen, gelatin, dermal graft, processed dermal graft, dried acellular particulate dermal matrix, fat cells, stem cells, chondrocytes, salts thereof, esters thereof, hydrates thereof, derivatives thereof, solvates thereof, and any combination thereof.

13. The surgical suture of claim 12, wherein the dermal filler comprises hydroxyapatite, a salt thereof, an ester thereof, a hydrate thereof, a derivative thereof, a solvate thereof, and any combination thereof.

14. The surgical suture of claim 13, wherein the thread is biodegradable or bioabsorbable, and wherein the dermal filler is formulated in the shape of a plurality of particles having an average diameter of 10 to 100 microns evenly distributed along at least one section of the thread.

15. A surgical suture according to any one of claims 1 to 14, for use in a cosmetic or therapeutic method of reducing the depth, volume or visibility of an undesired line, area, wrinkle, depressed scar or fold in the skin of a subject in need thereof.

## Patentansprüche

1. Chirurgisches Nahtmaterial, umfassend:
(i) wenigstens eine flexible feste Matrix, die in der Form eines Fadens formuliert ist, wobei der Faden eine ultimative Zugfestigkeit aufweist, die ausreicht, um den Faden durch eine menschliche Haut oder Subkutis zu ziehen und den Faden, sobald er in der Haut oder Subkutis ist, zu manipulieren, derart, dass die Integrität des Fadens nicht wesentlich beeinträchtigt wird; wobei die wenigstens eine flexible feste Matrix in Assoziation mit:
(ii) wenigstens einem dermalen Filler steht, wobei der dermale Filler chemisch unterschiedlich von der flexiblen festen Matrix ist; und wobei der dermale Filler so konfiguriert ist, dass er sich allmählich von der flexiblen festen Matrix löst, während er in der Haut oder der Subkutis ist;
wobei wenigstens ein Abschnitt des Fadens in der Form ist von:
- einem gerillten Faden, wobei die Rille wenigstens teilweise mit dem dermalen Filler gefüllt ist; oder
- einem porösen Faden, wobei die Poren wenigstens teilweise mit dem dermalen Filler gefüllt sind; oder
- einem hohlen Faden, wenigstens teilweise mit dem dermalen Filler gefüllt.

2. Chirurgisches Nahtmaterial nach Anspruch 1, wobei wenigstens ein Abschnitt des Fadens wenigstens teilweise äußerlich mit dem dermalen Filler beschichtet ist.

3. Chirurgisches Nahtmaterial nach einem der Ansprüche 1 oder 2, ferner umfassend ein Mittel, das die Ablösung des dermalen Fillers von dem Faden fördert, während er in der Haut oder der Subkutis ist; wobei das Mittel ein hygroskopisches, diffusibles oder osmotisches Mittel ist.

4. Chirurgisches Nahtmaterial nach einem der Ansprüche 1 bis 3, wobei der dermale Filler im Wesentlichen gleichmäßig und homogen entlang wenigstens eines Abschnitts des Fadens verteilt ist.

5. Chirurgisches Nahtmaterial nach einem der Ansprüche 1 bis 4, wobei wenigstens eine der folgenden Eigenschaften zutrifft:
- der Faden ist biologisch abbaubar oder bioresorbierbar;
- der Faden ist nicht biologisch abbaubar oder nicht bioresorbierbar.

6. Chirurgisches Nahtmaterial nach einem der Ansprüche 1 bis 5, wobei der dermale Filler wenigstens einen flüssigen dermalen Filler oder wenigstens einen halbfesten dermalen Filler umfasst.

7. Chirurgisches Nahtmaterial nach Anspruch 6, wobei der dermale Filler als Flüssigkeit oder als Halbfeststoff formuliert ist.

8. Chirurgisches Nahtmaterial nach einem der Ansprüche 1 bis 7, wobei der dermale Filler wenigstens einen starren dermalen Filler umfasst.

9. Chirurgisches Nahtmaterial nach Anspruch 8, wobei der starre dermale Filler aufweist:
(i) eine ultimative Zugfestigkeit von bis zu 300 MPa;
(ii) eine Druckfestigkeit von bis zu 1000 MPa;
(iii) einen Elastizitätsmodul von wenigstens 80 GPa;
(iv) eine Bruchzähigkeit von bis zu 1 MPa.m^{-1/2}; oder
(v) eine beliebige Kombination von (i) bis (iv).

10. Chirurgisches Nahtmaterial nach Anspruch 8 oder Anspruch 9, wobei der starre dermale Filler eine Keramik, ein Mineral oder ein Mineraloid ist.

11. Chirurgisches Nahtmaterial nach einem der Ansprüche 1 bis 10, wobei der starre dermale Filler in der Form einer Vielzahl von Partikeln formuliert ist; wobei der durchschnittliche Durchmesser der Partikel in der Vielzahl von Partikeln 5 bis 500 Mikrometer beträgt.

12. Chirurgisches Nahtmaterial nach einem der Ansprüche 1 bis 11, wobei der dermale Filler aus der Gruppe ausgewählt ist, die aus einem Apatit, Hyaluronsäure, Polycaprolacton, Polymethylmethacrylat, Silikon, Polylactidsäure, Kollagen, Gelatine, dermalem Transplantat, verarbeitetem dermalem Transplantat, getrockneter azellulärer partikulärer dermaler Matrix, Fettzellen, Stammzellen, Chondrozyten, Salzen davon, Estern davon, Hydraten davon, Derivaten davon, Solvaten davon und einer beliebigen Kombination davon besteht.

13. Chirurgisches Nahtmaterial nach Anspruch 12, wobei der dermale Filler Hydroxyapatit, ein Salz davon, einen Ester davon, ein Hydrat davon, ein Derivat davon, ein Solvat davon und eine beliebige Kombination davon umfasst.

14. Chirurgisches Nahtmaterial nach Anspruch 13, wobei der Faden biologisch abbaubar oder bioresorbierbar ist und wobei der dermale Filler in der Form einer Vielzahl von Partikeln formuliert ist, die einen durchschnittlichen Durchmesser von 10 bis 100 Mikrometern aufweisen und gleichmäßig entlang wenigstens eines Abschnitts des Fadens verteilt sind.

15. Chirurgisches Nahtmaterial nach einem der Ansprüche 1 bis 14 zur Verwendung in einem kosmetischen oder therapeutischen Verfahren zur Reduzierung der Tiefe, des Volumens oder der Sichtbarkeit einer unerwünschten Linie, eines Bereichs, einer Falte, einer eingesunkenen Narbe oder einer Falte in der Haut eines entsprechenden Subjekts.

## Revendications

1. Suture chirurgicale, comprenant :
(i) au moins une matrice solide flexible formulée sous la forme d'un fil, le fil ayant une résistance à la traction ultime suffisante pour tirer le fil à travers une peau humaine ou un hypoderme humain et manipuler le fil une fois dans la peau ou l'hypoderme de telle sorte que l'intégrité du fil ne soit essentiellement pas compromise ; l'au moins une matrice solide flexible étant associée à :
(ii) au moins un produit de comblement dermique ; où le produit de comblement dermique est chimiquement différent de la matrice solide flexible ; et où le produit de comblement dermique est configuré pour se dissocier progressivement de la matrice solide flexible, lorsqu'il se trouve dans la peau ou l'hypoderme ;
où au moins une section du fil est formulée sous la forme :
• d'un fil rainuré, où la rainure est au moins partiellement remplie par le produit de comblement dermique ; ou
• d'un fil poreux, où les pores sont au moins partiellement remplis par le produit de comblement dermique ; ou
• un fil creux, au moins partiellement rempli par le produit de comblement dermique.

2. Suture chirurgicale selon la revendication 1, où au moins une section du fil est au moins partiellement recouverte à l'extérieur par le produit de comblement dermique.

3. Suture chirurgicale selon l'une quelconque des revendications 1 ou 2, comprenant en outre un agent qui favorise la dissociation du produit de comblement dermique du fil, lorsqu'il se trouve dans la peau ou l'hypoderme; où l'agent est un agent hygroscopique, diffusible ou osmotique.

4. Suture chirurgicale selon l'une quelconque des revendications 1 à 3, où le produit de comblement dermique est réparti de manière essentiellement uniforme et homogène le long d'au moins une section du fil.

5. Suture chirurgicale selon l'une quelconque des revendications 1 à 4, où au moins l'une des conditions suivantes est remplie :
• le fil est biodégradable ou bioabsorbable ;
• le fil est non biodégradable ou non bioabsorbable.

6. Suture chirurgicale selon l'une quelconque des revendications 1 à 5, où le produit de comblement dermique comprend au moins un produit de comblement dermique liquide ou au moins un produit de comblement dermique semi-solide.

7. Suture chirurgicale selon la revendication 6, où le produit de comblement dermique est formulé sous la forme d'un liquide ou est formulé sous la forme d'un semi-solide.

8. Suture chirurgicale selon l'une quelconque des revendications 1 à 7, où le produit de comblement dermique comprend au moins un produit de comblement dermique rigide.

9. Suture chirurgicale selon la revendication 8, où le produit de comblement dermique rigide présente :
(i) une résistance à la traction ultime allant jusqu'à 300 MPa ;
(ii) une résistance à la compression allant jusqu'à 1 000 MPa ;
(iii) un module d'élasticité d'au moins 80 GPa ;
(iv) une ténacité à la rupture allant jusqu'à 1 MPa.m^{-1/2} ; ou
(v) toute combinaison de (i) à (iv).

10. Suture chirurgicale selon la revendication 8 ou la revendication 9, où le produit de comblement dermique rigide est une céramique, un minéral ou un minéraloïde.

11. Suture chirurgicale selon l'une quelconque des revendications 1 à 10, où le produit de comblement dermique rigide est formulé sous la forme d'une pluralité de particules ; où le diamètre moyen des particules dans la pluralité de particules est de 5 à 500 microns.

12. Suture chirurgicale selon l'une quelconque des revendications 1 à 11, où le produit de comblement dermique est choisi dans le groupe constitué par une apatite, un acide hyaluronique, une polycaprolactone, un polyméthacrylate de méthyle, une silicone, un acide polylactique, un collagène, une gélatine, une greffe dermique, une greffe dermique traitée, une matrice dermique particulaire acellulaire séchée, des cellules adipeuses, des cellules souches, des chondrocytes, des sels de ceux-ci, des esters de ceux-ci, des hydrates de ceux-ci, des dérivés de ceux-ci, des solvates de ceux-ci et toute combinaison de ceux-ci.

13. Suture chirurgicale selon la revendication 12, où le produit de comblement dermique comprend de l'hydroxyapatite, un sel de celle-ci, un ester de celle-ci, un hydrate de celle-ci, un dérivé de celle-ci, un solvate de celle-ci et toute combinaison de ceux-ci.

14. Suture chirurgicale selon la revendication 13, où le fil est biodégradable ou bioabsorbable, et où le produit de comblement dermique est formulé sous la forme d'une pluralité de particules ayant un diamètre moyen de 10 à 100 microns réparties uniformément le long d'au moins une section du fil.

15. Suture chirurgicale selon l'une quelconque des revendications 1 à 14, pour utilisation dans un procédé cosmétique ou thérapeutique de réduction de la profondeur, du volume ou de la visibilité d'une ligne, d'une zone, d'une ride, d'une cicatrice déprimée ou d'un pli indésirable dans la peau d'un sujet qui en a besoin.
